(19) Europäisches Patentamt
European Patent Office
Office européen des brevets

(11) EP 1 982 979 B1

(12) EUROPÄISCHE PATENTSCHRIFT

(45) Veröffentlichungstag und Bekanntmachung des Hinweises auf die Patenterteilung:
**27.01.2010 Patentblatt 2010/04**

(51) Int Cl.:
***C07D 229/00*** *(2006.01)*

(21) Anmeldenummer: **08006854.7**

(22) Anmeldetag: **04.04.2008**

(54) **Herstellung uretdiongruppenhaltiger Polyisocyanate**

Manufacture of polyisocyanates containing uretdione groups

Fabrication de polyisocyanates présentant des groupes uretdiones

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **17.04.2007 DE 102007018015**

(43) Veröffentlichungstag der Anmeldung:
**22.10.2008 Patentblatt 2008/43**

(73) Patentinhaber: **Bayer MaterialScience AG**
**51368 Leverkusen (DE)**

(72) Erfinder:
- **Richter, Frank, Dr.**
  **51373 Leverkusen (DE)**
- **Halpaap, Reinhard, Dr.**
  **51519 Odenthal (DE)**

(56) Entgegenhaltungen:
**EP-A- 1 422 223** **EP-A- 1 533 301**
**US-A- 2 671 082** **US-A1- 2002 028 930**

EP 1 982 979 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft die Verwendung von speziellen Phosphinen als Katalysatoren für die Isocyanatdimerisierung (Uretdionbildung) und ein Verfahren zur Herstellung hoch uretdiongruppenhaltiger Polyisocyanate.

Uretdiongruppen aufweisende aliphatische Isocyanate auf Basis ggf. verzweigter, linearaliphatischer Diisocyanate zeichnen sich durch eine besonders niedrige Viskosität aus. Produkte auf Basis cycloaliphatischer Diisocyanate sind in der Regel hochviskose bis feste Substanzen, die als abspalterfreie, intern blockierte Vernetzer in Beschichtungssystemen eingesetzt werden können.

Eine Übersicht zur Isocyanat-Oligomerisierung wird in J. Prakt. Chem./Chem. Ztg. 1994, 336, 185- -200 gegeben.

Tris(dialkylamino)phosphine (DE-A 3 030 513) ggf. in Verbindung mit Cokatalysatoren (DE-A 3 437 635) weisen eine gute Selektivität für die Bildung von Uretdiongruppen (Uretdionselektivität) auf. Ihrer technischen Einsetzbarkeit steht allerdings der schwerwiegende Makel des hohen krebserzeugenden Potenzials ihrer Phosphoroxide, z.B. Hexamethylphosphorsäuretriamid, entgegen.

DE-A 3 739 549 offenbart die katalytische NCO-Dimerisierung mit 4-Dialkylaminopyridinen, wie z.B. 4-Dimethylaminopyridin (DMAP), wobei allerdings nur im Fall spezieller cycloaliphatischer Isocyanate wie Isophorondiisocyanat (IPDI) die Uretdionbildung selektiv verläuft. Linearaliphatische Isocyanate wie Hexamethylendiisocyanat (HDI) sowie verzweigte, linearaliphatische Isocyanate wie Trimethylhexandiisocyanat (TMDI) und Methylpentandiisocyanat (MPDI) liefern mit DMAP und verwandten Verbindungen hauptsächlich stark gefärbte, heterogene Reaktionsprodukte.

DE-A 1 670 720 offenbart die Herstellung von Uretdiongruppen aufweisenden aliphatischen Polyisocyanaten, wobei als Katalysatoren tertiäre Phosphine mit mindestens einem aliphatischen Substituenten sowie Bortrifluorid und seine Addukte eingesetzt werden. Es wird darauf hingewiesen, dass nur bei niedrigen Umsätzen und Reaktionstemperaturen zwischen 50 und 80°C hohe Anteile an Uretdiongruppen im Produkt erhalten werden können, wobei gleichzeitig Isocyanat-Trimere (Isocyanurate und Iminooxadiazindione) und, insbesondere bei höherer Temperatur, auch andere Nebenprodukte wie Carbodiimide oder Uretonimine gebildet werden. Uretonimine sind ganz besonders störend, da sie bei Lagerung zur Freisetzung von monomerem Isocyanat neigen.

Die DE-A 10254878 beschreibt die Verwendung von Phosphinen, die mindestens einen cycloaliphatischen, P-gebundenen Rest aufweisen als Katalysatoren für die NCO-Dimerisierung. Diese Katalysatoren zeichnen sich durch eine wesentlich höhere Uretdionselektivität verglichen mit anderen Trialkylphosphinen des Standes der Technik aus. Den Einsatz eines Spezialfalls dieser Phosphine, bicyclische Reste aufweisende Vertreter, für den gleichen Einsatz wird in DE 10354544 beschrieben.

Überraschenderweise wurde nun gefunden, dass Phosphine, die mindestens einen direkt Phosphorgebundenen, tertiären Alkylrest aufweisen, ebenfalls gut als Katalysatoren für die selektive Uretdion-Bildung (Isocyanat-Dimerisierung) geeignet sind.

Gegenstand der Erfindung ist die Verwendung von Phosphinen, die einen oder zwei direkt Phosphor-gebundene, tertiäre Alkylreste aufweisen, bei der Uretdion-Bildung (Isocyanat-Dimerisierung).

"Direkt Phosphor-gebundene, tertiäre Alkylreste" bedeutet, dass das direkt an den Phosphor gebundene Kohlenstoffatom ein tertiäres C-Atom ist, also ein solches, dass neben der C-P-Bindung noch Einfachbindungen zu drei weiteren C-Atomen aufweist.

Bevorzugte Phosphine zur Isocyanat-Dimerisierung entsprechen der Formel I:

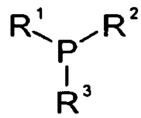

Formel I

wobei

- $R^1$: ein ggf. ein oder mehrfach $C_1$-$C_{12}$ alkyl- oder alkoxy-substituierter, tertiärer Alkylrest ist, wobei das tertiäre Kohlenstoffatom mit dem Phosphoratom durch eine kovalente (Einfach)bindung verknüpft ist
- $R^2$: für einen Rest aus der Gruppe der primären oder sekundären, ein oder mehrfach $C_1$-$C_{12}$ alkyl- oder alkoxy-substituierten, ggf. verzweigten, ggf. cycloaliphatischen $C_1$-$C_{20}$-Reste steht, mit der Maßgabe, dass das mit dem P-Atom verbundene Kohlenstoffatom mindestens ein Wasserstoffatom trägt und
- $R^3$: $R^1$ oder $R^2$ entspricht.

Bevorzugte Verbindungen der Formel I sind solche, in denen $R^1$ für tert.-Butyl (2-Methyl-prop-2-yl-), tert.-Amyl (2-Methyl-but-2-yl-) oder Adamantyl (Tricyclo[3.3.1.1]dec-1-yl-) steht.

Beispiele erfindungsgemäß einzusetzender Phosphine sind: Tert.-butyl-dimethylphosphin, Tert.-butyl-diethylphosphin, Tert.-butyl-di-n-propylphosphin, Tert.-butyl-di-isopropylphosphin, Tert.-butyl-dibutylphosphin wobei Butyl' für die Isomeren, n-Butyl, iso-Butyl, 2-Butyl sowie cycloButyl jedoch nicht tert-Butyl stehen kann, Tert.-butyl-dihexylphosphin (alle isomeren Hexylreste die keine direkt P-gebundenen tert.-C-Atome aufweisen), Tert.-butyl-dioctylphosphin (alle isomeren Octylreste die keine direkt P-gebundenen tert.-C-Atome aufweisen), Di-tert.-butyl-methylphosphin, Di-tert.-butyl-ethylphosphin, Di-tert.-butyl-n-propylphosphin, Di-tert.-butyl-isopropylphosphin, Di-tert.-butyl-butylphosphin (alle isomeren Butylreste die keine direkt P-gebundenen tert.-C-Atome aufweisen, s. oben), Di-tert.-butyl-hexylphosphin (alle isomeren Hexylreste die keine direkt P-gebundenen tert.-C-Atome aufweisen), Di-tert.-butyl-octylphosphin (alle isomeren Octylreste die keine direkt P-gebundenen tert.-C-Atome aufweisen), Tert.-amyl-dimethylphosphin, Tert.-amyl-diethylphosphin, Tert.-amyl-di-n-propylphosphin, Tert.-amyl-di-isopropylphosphin, Tert.-amyl-dibutylphosphin (alle isomeren Butylreste die keine direkt P-gebundenen tert.-C-Atome aufweisen), Tert.-amyl-dihexylphosphin (alle isomeren Hexylreste die keine direkt P-gebundenen tert.-C-Atome aufweisen), Tert.-amyl-dioctylphosphin (alle isomeren Octylreste die keine direkt P-gebundenen tert.-C-Atome aufweisen), Di-tert.-amyl-methylphosphin, Di-tert.-amyl-ethylphosphin, Di-tert.-amyl-n-propylphosphin, Di-tert.-amyl-isopropylphosphin, Di-tert.-amyl-butylphosphin (alle isomeren Butylreste die keine direkt P-gebundenen tert.-C-Atome aufweisen), Di-tert.-amyl-hexylphosphin (alle isomeren Hexylreste die keine direkt P-gebundenen tert.-C-Atome aufweisen), Di-tert.-amyl-octylphosphin (alle isomeren Octylreste die keine direkt P-gebundenen tert.-C-Atome aufweisen), Adamantyldimethylphosphin, Adamantyldiethylphosphin, Adamantyldi-n-propylphosphin, Adamantyldi-isopropylphosphin, Adamantyldibutylphosphin (alle isomeren Butylreste die keine direkt P-gebundenen tert.-C-Atome aufweisen, s. oben), Adamantyldihexylphosphin (alle isomeren Hexylreste die keine direkt P-gebundenen tert.-C-Atome aufweisen), Adamantyldioctylphosphin (alle isomeren Octylreste die keine direkt P-gebundenen tert.-C-Atome aufweisen), Diadamantylmethylphosphin, Diadamantylethylphosphin, Diadamantyl-n-propylphosphin, Diadamantylisopropylphosphin, Diadamantylbutylphosphin (alle isomeren Butylreste die keine direkt P-gebundenen tert.-C-Atome aufweisen), Diadamantylhexylphosphin (alle isomeren Hexylreste die keine direkt P-gebundenen tert.-C-Atome aufweisen) sowie Diadamantyloctylphosphin (alle isomeren Octylreste die keine direkt P-gebundenen tert.-C-Atome aufweisen).

Diese können für die Uretdionbildung einzeln, in beliebigen Mischungen untereinander oder in Mischungen mit anderen primären, sekundären und/oder tertiären Alkyl-, Aralkyl- und/oder A-rylphosphinen verwendet werden.

Ein weiterer Gegenstand der Erfindung ist ein Verfahren zur Dimerisierung von Isocyanaten, bei dem

a) mindestens ein organisches Isocyanat,

b) ein Katalysator enthaltend mindestens ein Phosphin, das einen oder zwei direkt Phosphor-gebundene, tertiäre Alkylreste aufweist,

c) optional Lösemittel und

d) optional Additive

zur Reaktion gebracht werden.

Die Menge des im erfindungsgemäßen Verfahren einzusetzenden Katalysators richtet sich in erster Linie nach dem verwendeten Isocyanat und der angestrebten Reaktionsgeschwindigkeit und liegt im Bereich 0,01 bis 10 mol-%, bezogen auf die Summe der Stoffmengen des eingesetzten Isocyanates und des Katalysators. Bevorzugt werden 0,05 bis 5 mol-% Katalysator eingesetzt.

Der Katalysator b) kann im erfindungsgemäßen Verfahren unverdünnt oder in Lösungsmitteln gelöst eingesetzt werden. Als Lösungsmittel kommen dabei alle Verbindungen in Frage, die nicht mit Phosphinen reagieren wie z.B. aliphatische oder aromatische Kohlenwasserstoffe, Alkohole, Ketone, Ester sowie Ether. Bevorzugt werden die Phosphine im erfindungsgemäßen Verfahren unverdünnt eingesetzt.

Als in a) erfindungsgemäß einzusetzende Isocyanate können prinzipiell alle bekannten, durch Phosgenierung oder nach phosgenfreien Verfahren hergestellten organischen Isocyanate einzeln oder in beliebigen Mischungen untereinander verwendet werden.

Bevorzugt ist die Verwendung von aliphatischen, cycloaliphatischen oder araliphatischen Di- oder Polyisocyanaten einer NCO-Funktionalität ≥ 2.

Besonders bevorzugt ist die Verwendung ggf. verzweigter, ggf. cyclische Reste enthaltender aliphatischer Diisocyanate mit an ein primäres Kohlenstoffatom gebundenen Isocyanatgruppen. Beispiele hierfür sind Butandiisocyanat, Pentandiisocyanat, Hexandiisocyanat, Heptandiisocyanat, Octandiisocyanat, Nonandiisocyanat, Dekandiisocyanat, Undekandiisocyanat und Dodecandiisocyanat, wobei beliebige Isomere der vorstehend genannten Verbindungen zum

Einsatz kommen können.

Ganz besonders bevorzugt werden Hexamethylendiisocyanat (HDI), Methylpentandiisocyanat (MPDI), Trimethylhexandiisocyanat (TMDI), Bis(isocyanatomethyl)cyclohexan ($H_6XDI$) sowie Norbornandiisocyanat (NBDI) einzeln oder in beliebigen Mischungen untereinander eingesetzt.

Darüber hinaus können Isophorondiisocyanat (IPDI), Bis(isocyanatocyclohexyl)methan ($H_{12}MDI$), Bis(isocyantomethyl)benzol (Xylylendiisocyanat, XDI) und Bis(2-isocyantoprop-2-yl)benzol (Tetramethylxylylendiisocyanat, TMXDI) im erfindungsgemäßen Verfahren eingesetzt werden.

Das erfindungsgemäße Verfahren wird so geführt, dass der Umsatz der NCO-Gruppen, bevorzugt von 5 bis 90 mol-%, insbesondere 10 bis 60 mol-%, ganz besonders bevorzugt von 10 bis 50 mol-% beträgt.

Das erfindungsgemäße Verfahren wird üblicherweise im Temperaturbereich 0°C bis 150°C durchgeführt.

Um Umsätze der NCO-Gruppen gemäß der vorstehenden Bereiche zu realisieren, wird die Reaktion bei dem gewünschten Umsetzungsgrad abgebrochen.

Zum Abbruch der Reaktion nach Erreichen des gewünschten Umsetzungsgrades eignen sich prinzipiell alle vorbeschriebenen Katalysatorgifte (DE-A 1670667, 1670720, 1934763, 1954093, 3437635, US 4614785) wie Alkylierungsmittel (z.B. Dimethylsulfat, Toluolsulfonsäuremethylester), organische oder anorganische Peroxide, Säurechloride sowie Schwefel, die mit dem Katalysator ggf. unter Temperaturerhöhung zur Reaktion gebracht werden (Variante A, vgl. auch Beispiele 1 bis 6).

Nach der Desaktivierung der Reaktionsmischung nach Variante A kann nicht umgesetztes Monomer und/oder der deaktivierte Katalysator abgetrennt werden.

Das Verfahren kann auch ohne chemische Deaktivierung des Katalysators abgebrochen werden. Dazu wird unmittelbar nach Erreichen des gewünschten Umsatzes der aktive Katalysator aus der Reaktionsmischung abgetrennt, um eine Weiterreaktion ggf. unter Nebenproduktbildung zu unterbinden. (Variante B).

Gleichzeitig mit oder auch nach der Katalysatorabtrennung kann nicht umgesetztes Restmonomer aus der nach Variante B behandelten Reaktionsmischung abgetrennt werden.

Im erfindungsgemäßen Verfahren können zur Abtrennung nicht umgesetzter Monomere, des Katalysators und/oder anderer unerwünschter Bestandteile aus der Reaktionsmischung alle bekannten Separationstechniken wie z.B. Destillation, Extraktion oder Kristallisation/Filtration verwendet werden. Bevorzugt ist die Destillation, ggf. in der speziellen Ausführungsform der Dünnschichtdestillation. Selbstverständlich können auch Kombinationen zweier oder mehrerer dieser Techniken angewendet werden.

Bevorzugt wird zum Reaktionsabbruch nach Variante B der Katalysator destillativ entfernt, wobei gleichzeitig ggf. nicht umgesetztes Monomer mit entfernt wird.

Bevorzugt wird bei der Aufarbeitung einer nach Variante A oder B abgebrochenen Reaktion das enthaltene Restmonomer destillativ entfernt.

Soll das erfindungsgemäß hergestellte Polyisocyanat noch freies, nicht umgesetztes Monomer enthalten, wie es z.B. für die Weiterverarbeitung zu NCO-blockierten Produkten oder NCO-armen bzw. -freien Polyuretdionhärtern z.B. für den Pulverlackbereich von Interesse ist, so kann nach Reaktionsabbruch (Varianten A und B) auf die Monomerenabtrennung verzichtet werden.

Es ist für die Durchführung des erfindungsgemäßen Verfahrens unerheblich, ob das Verfahren ganz oder teilweise diskontinuierlich oder kontinuierlich durchgeführt wird.

Weiterhin können im erfindungsgemäßen Verfahren zu einem beliebigen Zeitpunkt, in der Polyisocyanatchemie übliche Additive und Stabilisatoren zugesetzt werden. Beispiele sind Antioxidanzien, wie z.B. sterisch gehinderte Phenole (2,6-Di-tert.butylphenol, 4-Methyl-2,6-di-tert.butylphenol), Lichtschutzmittel, wie z.B. HALS-Amine, Triazole etc., schwache Säuren oder Katalysatoren für die NCO-OH-Reaktion wie z.B. Dibutylzinndilaurat (DBTL).

Des weiteren kann es sinnvoll sein, einem nach Variante B aufgearbeiteten Produkt, geringe Mengen eines in Variante A zu verwendenden Katalysatorgiftes zuzusetzen, um die Rückspaltstabilität zu erhöhen und die Neigung zur Nebenproduktbildung, zur Verfärbung bzw. zur Weiterreaktion der freien NCO-Gruppen untereinander, z.B. bei Produktlagerung, zu unterdrücken.

Nach dem erfindungsgemäßen Verfahren hergestellte Produkte auf Basis ggf. verzweigter, linearaliphatischer Di- oder Polyisocyanate, die keine Cycloalkylsubstituenten aufweisen, sind farbhell und haben eine Viskosität < 1000 mPas/23°C. Werden cycloaliphatische und/oder araliphatische Di- oder Polyisocyanate eingesetzt, werden hochviskose bis feste Harze erhalten (Viskosität > 10000 mPas/23°C).

In monomerenarmer Form, d.h. nach Abtrennung von nicht umgesetztem Monomer, weisen die erfindungsgemäßen Produkte einen NCO-Gehalt < 30 Gew.-%, bevorzugt < 25 Gew.-%, auf.

Die nach dem erfindungsgemäßen Verfahren hergestellten Polyisocyanate dienen als Ausgangsmaterialien zur Herstellung von z.B. Formkörpern (ggf. geschäumt), Lacken, Beschichtungsmitteln, Klebstoffen oder Zuschlagstoffen, wobei die enthaltenen freien, nicht uretdionisierten NCO-Gruppen auch ggf. blockiert sein können.

Zur Blockierung der freien, nicht uretdionisierten NCO-Gruppen eignen sich alle dem Fachmann bekannten Methoden. Als Blockierungsmittel können insbesondere Phenole (z.B. Phenol, Nonylphenol, Kresol), Oxime (z.B. Butanonoxim, Cyclohexanonoxim), Lactame (z.B. □-Caprolactam), sekundäre Amine (z.B. Diisopropylamin), Pyrazole (z.B. Dimethylpyrazol), Imidazole, Triazole) oder Malon- und Essigsäureester verwendet werden.

Die nach dem erfindungsgemäßen Verfahren hergestellten, weitgehend nebenproduktfreien, Uretdiongruppen aufweisenden Polyisocyanate können insbesondere zur Herstellung von Ein- und Zweikomponenten-Polyurethanlacken ggf. in Mischungen mit anderen Di- oder Polyisocyanaten des Standes der Technik, wie Biuret-, Urethan-, Allophanat-, Isocyanurat-, sowie Iminooxadiazindiongruppen enthaltenden Di- oder Polyisocyanaten eingesetzt werden.

Ebenfalls besonders bevorzugt ist die Verwendung der erfindungsgemäß hergestellten Polyisocyanate auf Basis ggf. verzweigter, linearaliphatischer Isocyanate als Reaktivverdünner zur Viskositätserniedrigung höherviskoser Polyisocyanat-Harze.

Zur Umsetzung der erfindungsgemäß hergestellten Polyisocyanate zum Polyurethan können alle Verbindungen mit mindestens zwei isocyanatreaktiven Funktionalitäten einzeln oder in beliebigen Mischungen untereinander (isocyanatreaktives Bindemittel) eingesetzt werden.

Bevorzugt ist die Verwendung eines oder mehrerer, in der Polyurethanchemie an sich bekannter, isocyanatreaktiver Bindemittel wie Polyhydroxyverbindungen oder Polyamine. Als Polyhydroxyverbindungen werden besonders bevorzugt Polyester-, Polyether-, Polyacrylat- und/oder Polycarbonsäure-Polyole, ggf. auch unter Zusatz niedermolekularer, mehrwertiger Alkohole eingesetzt.

Das Äquivalentverhältnis zwischen nicht uretdionisierter Isocyanatgruppe, die ggf. auch blockiert sein kann, und isocyanatreaktiver Funktionalität des isocyanatreaktiven Bindemittels, wie z.B. OH-, NH- oder COOH, liegt von 0,8 bis 3, vorzugsweise 0,8 bis 2.

Möglich ist der Einsatz eines Überschusses an isocyanatreaktivem Bindemittel, da die Spaltung des Uretdionringes ggf. bei erhöhter Temperatur und/oder Katalysatorzusatz zur Freisetzung weiterer NCO-Gruppen führt, die mit dem Überschuss an isocyanatreaktiven Funktionalitäten reagieren können. Dadurch erhöht sich die Netzwerkdichte des gebildeten Polymers und dessen Eigenschaften werden vorteilhaft beeinflusst.

Für die Beschleunigung der Vernetzungsreaktion der erfindungsgemäß hergestellten Polyisocyanate mit dem isocyanatreaktiven Bindemittel können alle aus der Polyurethanchemie bekannten Katalysatoren verwendet werden. Beispielweise können Metallsalze wie Dibutylzinn-IV-dilaurat, Zinn-II-bis(2-ethylhexanoat), Wismut-III-tris(2-ethylhexanoat), Zink-II-bis(2-ethylhexanoat) oder Zinkchlorid sowie tertiäre Amine wie 1,4-Diazabicyclo(2,2,2)oktan, Triethylamin oder Benzyldimethylamin verwendet werden.

Bei der Formulierung werden das erfindungsgemäß hergestellte, ggf. blockierte Polyisocyanat, das isocyanatreaktive Bindemittel, Katalysator(en) und ggf. die üblichen Zusätze wie Pigmente, Füllstoffe, Additive, Verlaufshilfsmittel, Entschäumer und/oder Mattierungsmittel miteinander auf einem üblichen Mischaggregat wie z.B. einer Sandmühle, ggf. unter Verwendung von Lösungsmitteln, vermischt und homogenisiert.

Als Lösungsmittel geeignet sind alle an sich bekannten üblichen Lacklösemittel wie z.B. Ethyl- und Butylacetat, Ethylen- oder Propylenglykolmono-methyl-, -ethyl- oder -propyletheracetat, 2-Butanon, 4-Methyl-2-pentanon, Cyclohexanon, Toluol, Xylol, Solventnaphtha, N-Methylpyrrolidon etc.

Die Beschichtungsmittel können in Lösung oder aus der Schmelze sowie ggf. in fester Form (Pulverlacke) nach den üblichen Methoden wie z.B. Streichen, Rollen, Gießen, Spritzen, Tauchen, dem Wirbelsinterverfahren oder durch elektrostatische Sprühverfahren auf dem zu beschichtenden Gegenstand appliziert werden.

Als Substrate eignen sich sämtliche bekannten Werkstoffe, insbesondere Metalle, Holz, Kunststoffe und Keramik.

## Beispiele:

Die Prozentangaben des Umsatzes berechnen sich durch Division der Menge an erhaltenem Produkt (Polyisocyanatharz) durch die Gesamtmenge der eingesetzten Edukte (Diisocyanat-Monomer und Katalysator) multipliziert mit 100. Alle weiteren Prozentangaben sind, soweit nicht anders vermerkt, als Gewichtsprozente zu verstehen.

Die dynamischen Viskositäten wurden bei 23°C mit dem Viskosimeter VT 550, Fa. Haake, Karlsruhe bestimmt. Durch Messungen bei unterschiedlichen Schergeschwindigkeiten wurde sichergestellt, dass das Fließverhalten der beschriebenen erfindungsgemäß hergestellten Polyisocyanate wie auch das der Vergleichsprodukte dem idealer Newtonscher Flüssigkeiten entspricht. Die Angabe der Schergeschwindigkeit kann deshalb entfallen.

Die Angaben 'mol-%' bzw. molares Verhältnis unterschiedlicher Strukturtypen zueinander' basieren auf NMR-spektroskopischen Messungen. Sie beziehen sich immer, wenn nicht anders angegeben, auf die Summe der durch die Modifizierungsreaktion (Oligomerisierung) aus den vorher freien NCO-Gruppen des zu modifizierenden Isocyanates gebildeten Strukturtypen. $^{13}$C-NMR-Messungen erfolgten auf den Geräten DPX 400, AVC 400 bzw. DRX 700 der Fa. Bruker, Karlsruhe, DE an ca. 50 %-igen Proben in trockenem $CDCl_3$ bzw. an ca. 80 %-igen Proben in $D_6$-DMSO ($^{13}$C-NMR: 100 bzw 176 MHz, relaxation delay: 4 sec, mind. 2000 scans). Als Referenz für die ppm-Skala wurden geringe Mengen Tetramethylsilan im entsprechenden Lösungsmittel ($\delta$ = 0 ppm) oder das Lösungsmittel alleine ($\delta$ = 77,0 ppm ($CDCl_3$) bzw. $\delta$ = 43,5 ppm ($D_6$-DMSO)) gewählt.

Soweit nicht anders angegeben, wurden die Reaktionen mit frisch entgastem HDI als Edukt durchgeführt. Die Bezeichnung frisch entgast' bedeutet dabei, dass das eingesetzte HDI unmittelbar vor der katalytischen Umsetzung durch mindestens 30-minütiges Rühren im Vakuum (< 1 mbar) von gelösten Gasen befreit und anschließend mit Stickstoff belüftet wurde.

Alle Reaktionen wurden unter einer Atmosphäre von trockenem Stickstoff durchgeführt.

Tert.-butyl substituierte Phosphine wurden nach literaturbekannten Methoden (K. Sasse in Methoden der organ. Chemie (Houben-Weyl) 4. Aufl., Bd. XII/1, Georg Thieme Verlag, Stuttgart, 1963) aus den entsprechenden Chlorphosphinen und Alkylierungsmitteln wie Alkyllithium oder Alkylmagnesiumhalogeniden (Grignard-Verbindungen) hergestellt, wie nachfolgend an einem Beispiel verdeutlich wird.

**Herstellung eines Katalysators** (nicht erfindungsgemäß)

Tert.-butyl-di-n-butylphosphin ($^tBuP^nBu_2$)

In einen 100 ml Rundkolben wurden bei Zimmertemperatur unter Stickstoff 10,3 g (65 mmol) Tert.butyldichlorphospin (Fa. Aldrich, 82018 Taufkirchen, DE) vorgelegt, in 20 ml Diethylether gelöst und unter Rühren auf -20°C gekühlt. Anschließend wurden tropfenweise 55 ml einer 2,5 M Lösung von n-Butyllithium in n-Hexan (Fa. Aldrich) zugesetzt, wobei sofort unter beträchtlicher Wärmeentwicklung ein weißer Feststoff ausfiel. Nach vollständiger Zugabe wurde langsam auf Zimmertemperatur erwärmt, anschließend eine Stunde am Rückfluss gekocht und nach Abkühlen auf Zimmertemperatur mit 10 ml 10%iger, sauerstofffreier, wässriger HCl versetzt wobei sich zwei klare, farblose Phasen bildeten. Nach Phasentrennung wurde die organische Phase destillativ bei Normaldruck vom größten Teil des Lösungsmittels befreit, anschließend filtriert und das Filtrat im Vakuum destilliert. Dabei wurden 7,4 g (56 % d. Theorie) $^tBuP^nBu_2$, Kp: 75°C bei 0,2 mbar, erhalten.

Di-1-adamantyl-n-butylphosphin wurde von der Fa. Strem, 77672 Kehl, DE, bezogen, 1-Adamantyl-di-n-butylphosphin nach o.g. Verfahren ausgehend von Adamantylmagnesiumbromid (J. Org. Chem. 47 1982 4120-4128) und Chlor-di-n-butylphosphin (Fa. Aldrich, 82018 Taufkirchen, DE) dargestellt.

**Beispiele 1 bis 5, erfindungsgemäß**

Jeweils 10 g frisch entgastes HDI wurden in mit Septen verschlossenen Glasgefäßen unter Stickstoff in Gegenwart der in Tabellen 1 bis 5 angegebenen Mengen des dort angegebenen Katalysators bei den angegebenen Temperaturen mit einem Magnetrührkern gerührt, wobei in regelmäßigen Abständen der Fortgang der Reaktion durch Messung des Brechungsindex' (bei 20°C und der Frequenz des Lichtes der D-Linie des Natriumspektrums, $n_D^{20}$) der Reaktionsmischung überprüft wurde. Der unmittelbar nach Homogenisierung von Katalysator und HDI gemessene Brechungsindex diente dabei als Referenz für den Startpunkt der Reaktion (Umsatz = 0; $n_D^{20}{}_{Start}$).

Zwischen den Größen Umsatz (Ausbeute) und $n_D^{20}$ der Reaktionsmischung besteht im Ausbeutebereich bis ca. 60 % Uretdion-Polyisocyanatharz in der Reaktionsmischung ein nahezu linearer Zusammenhang entsprechend folgender Formel:

$$\text{Umsatz [\%]} = 19{,}85 * n_D^{20} - 28{,}74$$

(vgl. Deutsche Offenlegungsschrift 103 54 544).

Der $n_D^{20}$ ist dabei der Wert für den Brechungsindex, der unter Berücksichtigung des o.g. Referenzwertes für den ‚HDI- und HDI-Oligomer-Anteil' gemäß folgender Formel erhalten wurde:

$$n_D^{20} = \text{Messwert} - (n_D^{20}{}_{Start} - n_D^{20}{}_{HDI}).$$

Die in Tab. 1 - 5 angegebenen Werte für den Umsatz sind auf Basis der o.g. Zusammenhänge aus den gemessenen Brechungsindizes ermittelt worden

Zur Bestimmung der Selektivität wurden die Umsatzproben zur Unterbindung der Weiterreaktion mit der ihrem Phosphingehalt entsprechenden Menge elementaren Schwefels versetzt und NMRspektroskopisch untersucht. Zur besseren Übersichtlichkeit der Selektivitäten wurde die Größe U/T als das molare Verhältnis der Uretdionstrukturen zur Summe der beiden Trimerstrukturen (Isocyanurat und Iminooxadiazindion) definiert.

**Tabelle 1:** Katalysator: $^tBuP^nBu_2$ (1,5 mol-%, bezogen auf HDI)

| Reaktionstemperatur: 30°C | |
|---|---|
| HDI-Umsatz [%] | U/T |
| 11% | **12** |
| 21% | **10** |
| 31% | **9** |
| 40% | **8** |
| 56% | **7** |
| 62% | **6** |

**Tabelle 2:** Katalysator: $^tBuP^nBu_2$ (0,6 mol-%, bezogen auf HDI)

| Reaktionstemperatur: 80°C | |
|---|---|
| HDI-Umsatz [%] | U/T |
| 8% | **27** |
| 17% | **17** |
| 35% | **8** |
| 41% | **6** |

**Tabelle 3:** Katalysator: $^tBu_2P^nBu$ (3,7 mol-%, bezogen auf HDI)

| Reaktionstemperatur: 80°C | |
|---|---|
| HDI-Umsatz [%] | U/T |
| 8% | **68** |
| 14% | **48** |
| 18% | **13** |
| 31% | **6** |
| 41% | **5** |

**Tabelle 4:** Katalysator: 1-Adamantyl-di-n-butylphosphin (1,4 mol-%, bezogen auf HDI)

| Reaktionstemperatur: 80°C | |
|---|---|
| HDI-Umsatz [%] | U/T |
| 10% | **17** |
| 20% | **13** |
| 45% | **5** |
| 52% | **4** |

**Tabelle 5:** Katalysator: Di-1-adamantyl-n-butylphosphin

| (2,3 mol-%, bezogen auf HDI) Reaktionstemperatur: 80°C | |
|---|---|
| HDI-Umsatz [%] | U/T |
| 6% | **48** |
| 20% | **14** |
| 30% | **9** |

**Beispiel 6 und 7 (Vergleichsbeispiele)**

Jeweils 10 g frisch entgastes HDI wurden in mit Septen verschlossenen Glasgefäßen unter Stickstoff in Gegenwart der in Tabellen 6 und 7 angegebenen Mengen des dort angegebenen Katalysators bei den angegebenen Temperaturen mit einem Magnetrührkern gerührt, wobei in regelmäßigen Abständen der Fortgang der Reaktion wie oben angegeben überprüft wird. Wie den Werten aus Tabellen 6 und 7 zu entnehmen ist, haben die erfindungsgemäßen Katalysatoren gegenüber ihren nächstliegenden Pendants mit mono- bzw. bicyclo-Alkylsubstituenten am Phosphor (mit sekundärem, P-gebundenem Kohlenstoffatom) deutliche Vorteile hinsichtlich der UretdionSelektivität bei gegebenem HDI-Umsatz.

**Tabelle 6**: Katalysator: Cyclohexyl-P-$^{n}Hex_2$ (0,5 mol-%, bezogen auf HDI)

| Reaktionstemperatur: 80°C, vgl. DE 102 54 878, Bsp. 1 | |
|---|---|
| HDI-Umsatz [%] | **U/T** |
| 15 | **7** |
| 35 | **4** |
| 45 | **3** |
| 60 | **2** |

**Tabelle 7:** Katalysator: 2-Norbornyl-P-$^{n}Hex_2$ (0,3 mol-%, bezogen auf HDI)

| Reaktionstemperatur: 80°C, vgl. DE 103 54 544, Bsp. 3 | |
|---|---|
| HDI-Umsatz [%] | **U/T** |
| 12 | **19** |
| 16 | **15** |
| 20 | **13** |
| 33 | **6** |
| 37 | **5** |
| 40 | **4** |

**Patentansprüche**

1. Verwendung von Phosphinen, die einen oder zwei direkt Phosphor-gebundene, tertiäre Alkylreste aufweisen, bei der Uretdion-Bildung (Isocyanat-Dimerisierung).

2. Verwendung gemäß Anspruch 1, **dadurch gekennzeichnet, dass** die Phosphine der Formel I entsprechen:

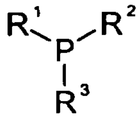

Formel I

wobei

$R^1$: ein ggf. ein oder mehrfach $C_1$-$C_{12}$ alkyl- oder alkoxy-substituierter, tertiärer Alkylrest ist, wobei das tertiäre Kohlenstoffatom mit dem Phosphoratom durch eine kovalente (Einfach)bindung verknüpft ist
$R^2$: für einen Rest aus der Gruppe der primären oder sekundären, ein oder mehrfach $C_1$-$C_{12}$ alkyl- oder alkoxy-substituierten, ggf. verzweigten, ggf. cycloaliphatischen $C_1$-$C_{20}$-Reste steht, mit der Maßgabe, dass das mit dem P-Atom verbundene Kohlenstoffatom mindestens ein Wasserstoffatom trägt und
$R^3$: $R^1$ oder $R^2$ entspricht.

3. Verwendung gemäß Anspruch 2, **dadurch gekennzeichnet, dass** in Formel I $R^1$ = tert.-Butyl-, tert.-Amyl oder Adamantyl ist.

4. Verwendung, gemäß einem der Ansprüche 1 bis 3, **dadurch gekennzeichnet, dass** für die Dimerisierung aliphatische, cycloaliphatische oder araliphatische Di- oder Polyisocyanate einer NCO-Funktionalität ≥ 2 eingesetzt werden.

5. Verfahren zur Dimerisierung von Isocyanaten, bei dem

a) mindestens ein organisches Isocyanat,
b) ein Katalysator enthaltend mindestens ein Phosphin, das einen oder zwei direkt Phosphor-gebundene, tertiäre Alkylreste aufweist,
c) optional Lösemittel und
d) optional Additive

zur Reaktion gebracht.

6. Verfahren gemäß Anspruch 5, **dadurch gekennzeichnet, dass** die Dimerisierung bei einer Temperatur von 0 bis 150°C bis zu einem Umsatz der NCO-Gruppen von 5 bis 90 mol-% geführt und danach abgebrochen wird.

7. Verfahren gemäß Anspruch 5 oder 6, **dadurch gekennzeichnet, dass** in Komponente a) aliphatische, cycloaliphatische oder araliphatische Di- oder Polyisocyanaten einer NCO-Funktionalität ≥ 2 eingesetzt werden.

## Claims

1. Use, in uretdione formation (isocyanate dimerization), of phosphines having one or two tertiary alkyl radicals directly bonded to phosphorus.

2. Use according to Claim 1, **characterized in that** the phosphines have formula I:

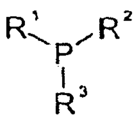

formula I

in which

$R^1$ is a tertiary alkyl radical optionally mono-substituted or polysubstituted by $C_1$-$C_{12}$-alkyl or $C_1$-$C_{12}$-alkoxy, the tertiary carbon atom being linked to the phosphorus atom by a covalent (single) bond,
$R^2$ is a radical from the group comprising primary or secondary, optionally branched, optionally cycloaliphatic $C_1$-$C_{20}$ radicals monosubstituted or polysubstituted by $C_1$-$C_{12}$-alkyl or $C_1$-$C_{12}$-alkoxy, with the proviso that the carbon atom bonded to the P atom carries at least one hydrogen atom, and
$R^3$ is $R^1$ or $R^2$.

3. Use according to Claim 2, **characterized in that**, in formula I, $R^1$ = tert-butyl, tert-amyl or adamantyl.

4. Use according to one of Claims 1 to 3, **characterized in that** aliphatic, cycloaliphatic or araliphatic diisocyanates or polyisocyanates with an NCO functionality of ≥ 2 are used for the dimerization.

5. Process for the dimerization of isocyanates, wherein

a) at least one organic isocyanate,
b) a catalyst containing at least one phosphine having one or two tertiary alkyl radicals directly bonded to phosphorus,
c) optionally solvents and
d) optionally additives

are reacted together.

6. Process according to Claim 5, **characterized in that** the dimerization is carried out at a temperature of 0 to 150°C until the conversion of the NCO groups is 5 to 90 mol%, and then stopped.

7. Process according to Claim 5 or 6, **characterized in that** aliphatic, cycloaliphatic or araliphatic diisocyanates or polyisocyanates with an NCO functionality of ≥ 2 are used in component a).

**Revendications**

1. Utilisation de phosphines présentant un ou deux restes alkyle tertiaire liés directement au phosphore, lors de la formation d'uretdiones (dimérisation d'isocyanates).

2. Utilisation selon la revendication 1, **caractérisée en ce que** les phosphines correspondent à la formule I

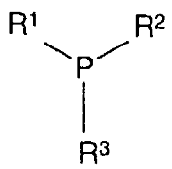

Formule I

où

$R^1$ : est un reste alkyle tertiaire substitué le cas échéant une ou plusieurs fois par un alkyle en $C_1$-$C_{12}$ ou un alcoxy, l'atome de carbone tertiaire étant relié à l'atome de phosphore par une liaison (simple) covalente
$R^2$ : représente un reste du groupe des restes en $C_1$-$C_{20}$ primaires ou secondaires, substitués une ou plusieurs fois par un alkyle en $C_1$-$C_{12}$ ou alcoxy, le cas échéant ratifié, le cas échéant cycloaliphatique, à la condition que l'atome de carbone lié à l'atome de P porte au moins un atome d'hydrogène et
$R^3$ : correspond à $R^1$ ou $R^2$.

3. Utilisation selon la revendication 2, **caractérisée en ce que** dans la formule I $R^1$ est un tert-butyle, tert-amyle ou adamantyle.

4. Utilisation selon l'une des revendications 1 à 3, **caractérisée en ce que**, pour la dimérisation, on utilise des di- ou polyisocyanates aliphatiques, cycloaliphatiques ou araliphatiques de fonctionnalité NCO ≥ 2.

5. Procédé de dimérisation d'isocyanates, dans lequel on met à réagir

a) au moins un isocyanate organique,
b) un catalysateur contenant au moins une phosphine qui présente un ou deux restes alkyle tertiaire liés directement au phosphore,
c) facultativement des solvants et
d) facultativement des additifs.

6. Procédé selon la revendication 5, **caractérisé en ce que** la dimérisation est effectuée à une température de 0 à 150°C jusqu'à un rendement en groupes NCO de 5 à 90 % en mole et est ensuite interrompue.

7. Procédé selon la revendication 5 ou 6, **caractérisé en ce qu'**on utilise comme composants a) des di- ou polyisocyanates aliphatiques, cycloaliphatiques ou araliphatiques de fonctionnalité NCO ≥ 2.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 3030513 A
- DE 3437635 A
- DE 3739549 A
- DE 1670720 A
- DE 10254878 A
- DE 10354544
- DE 1670667 A
- DE 1934763 A
- DE 1954093 A
- US 4614785 A
- DE 10254878


### In der Beschreibung aufgeführte Nicht-Patentliteratur


- *J. Prakt. Chem./Chem. Ztg.,* 1994, vol. 336, 185-200
- **K. SASSE.** Methoden der organ. Chemie. Georg Thieme Verlag, 1963, vol. XII/1
- *J. Org. Chem.,* 1982, vol. 47, 4120-4128